# EUROPÄISCHE PATENTSCHRIFT

(11) **EP 1 453 477 B1**
(45) Veröffentlichungstag und Bekanntmachung des Hinweises auf die Patenterteilung: **17.10.2007**
(21) Anmeldenummer: 02792851.4
(22) Anmeldetag: 02.12.2002
(51) Int. Cl.: A61K 8/41, A61Q 5/10

(54) **ENTWICKLER-KUPPLER-KOMBINATION MIT 2,4-DICHLOR-3-AMINOPHENOL**
DEVELOPER-COUPLER COMBINATION CONTAINING 2,4-DICHLORO-3-AMINOPHENOL
COMBINAISON REVELATEURS-COUPLEUR CONTENANT DU 2,4-DICHLORO-3-AMINOPHENOL

(30) Priorität: 11.12.2001 DE 10160814
(43) Veröffentlichungstag der Anmeldung: 08.09.2004
(73) Patentinhaber: Henkel Kommanditgesellschaft auf Aktien, 40589 Düsseldorf (DE)
(72) Erfinder: HÖFFKES, Horst, 40595 Düsseldorf (DE); ROSE, David, 40723 Hilden (DE)
(86) Internationale Anmeldenummer: PCT/EP2002/013587
(87) Internationale Veröffentlichungsnummer: WO 2003/053384

(56) Entgegenhaltungen:
- DE-A- 19 916 029
- DE-U- 20 013 155
- DE-U- 20 120 050

## Beschreibung

Die vorliegende Erfindung betrifft Mittel zum Färben von Keratinfasern, die 2,4-Dichlor-3-aminophenol als Kupplerkomponente in Verbindung mit einer speziellen Entwicklerkombination, bestehend aus mindestens einem p-Aminophenolderivat und mindestens einem p-Phenylendiaminderivat enthalten, ein Verfahren zum Färben von Keratinfasern mit diesen Mitteln sowie die Verwendung dieser Farbstoffkombinationen zur Färbung von Keratinfasern.

Keratinfasern, insbesondere das menschliche Haar werden heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel empfindlich, so daß eine vielfach unerwünschte Nuancenverschiebung oder gar eine sichtbare "Entfärbung" eintreten kann.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kuppler-komponenten die eigentlichen Farbstoffe aus. Die Oxidationsfärbemittel zeichnen sich durch hervorragende, lang anhaltende Färbeergebnisse aus.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme, Schweiß, Reibung und den Einfluß chemischer Reduktionsmittel, z.B. Dauerwellenflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie dennoch nicht den individuellen Wünschen der einzelnen Person entspricht und rückgängig gemacht werden soll.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffkomponenten, insbesondere für die Erzielung von Färbungen im Rotbraun- und Violettbereich.

Es wurde nunmehr überraschenderweise gefunden, daß besonders intensive Färbungen mit ausgezeichneten Echtheitseigenschaften und außerordentlich guten toxikologischen Eigenschaften erhalten werden können, wenn Färbemittel eingesetzt werden, die eine besondere Farbstoffkombination aus 2,4-Dichlor-3-aminophenol und mindestens zwei speziellen Entwicklerkomponenten enthalten.

Ein erster Gegenstand der vorliegenden Erfindung ist daher ein Mittel zum Färben von Keratinfasern, das in einem zum Färben geeigneten Medium als Oxidationsfarbstoffvorprodukte eine Kombination aus
a) 2,4-Dichlor-3-aminophenol,
b) mindestens einer Verbindung gemäß Formel (I) 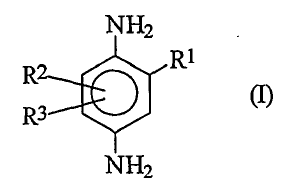 worin
   - der Rest R¹ steht für eine verzweigte oder unverzweigte C₁-C₄-Mono- oder eine C₂-C₆- Polyhydroxyalkylgruppe und
   - die beiden Reste R² und R³ stehen unabhängig voneinander für Wasserstoff, ein Halogenatom, eine C₁-C₄-Alkylgruppe, eine C₁-C₄-Mono- oder eine C₂-C₆-Polyhydroxyalkylgruppe, eine Amino-C₁-C₄-alkylgruppe, eine C₁-C₄-Alkoxygruppe, eine Hydroxy-C₁-C₄-alkoxygruppe oder für eine Gruppe OZ oder SZ, worin Z eine C₁-C₄-Alkylgruppe, eine C₁-C₄-Mono- oder eine C₂-C₆-Polyhydroxyalkylgruppe bedeutet, und
c) mindestens einer Verbindung gemäß Formel (II) 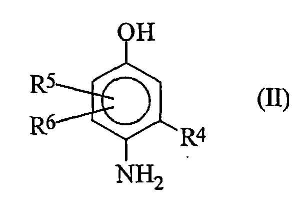 worin
   - der Rest R⁴ steht für eine verzweigte oder unverzweigte C₁-C₄-Alkylgruppe und
   - die beiden Reste R⁵ und R⁶ stehen unabhängig voneinander für Wasserstoff, Halogen, eine C₁-C₄-Alkylgruppe, eine C₁-C₄-Mono- oder eine C₂-C₆-Polyhydroxyalkylgruppe, eine C₁-C₄-Aminoalkylgruppe, eine C₁-C₄-Alkoxygruppe oder für eine C₁-C₄-Hydroxyalkoxygruppe
   oder den physiologisch verträglichen Salzen dieser Verbindungen enthält.

Unter Keratinfasern werden erfindungsgemäß Pelze, Wolle, Federn und insbesondere menschliche Haare verstanden.

Beispiele für physiologisch verträgliche Salze der erfindungsgemäßen Verbindungen sind die Hydrochloride, Hydrobromide, die Sulfate, die Phosphate, die Acetate, die Propionate, die Citrate und die Lactate.

Beispiele für die als Substituenten in den erfindungsgemäßen Verbindungen der Formel (I) und (II) genannten C₁- bis C₄-Alkylgruppen sind die Gruppen Methyl, Ethyl, Propyl, Isopropyl und Butyl. Ethyl und Methyl sind bevorzugte Alkylgruppen. Erfindungsgemäß bevorzugte C₁- bis C₄-Alkoxygruppen sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab.

Als erfindungsgemäß besonders geeignet haben sich p-Phenylendiamin-Derivate gemäß Formel (I) erwiesen, bei denen der Rest R¹ für eine C₂-Monohydroxyalkylgruppe steht, wobei es sich um eine α oder β-Monohydroxyalkylgruppe handeln kann.

Erfindungsgemäß bevorzugt sind auch p-Phenylendiaminderivate gemäß Formel (I), bei denen der Rest R¹ für eine C₂-Dihydroxyalkylgruppe steht.

Erfindungsgemäße Verbindungen gemäß der allgemeinen Formel (I), bei denen die Reste R² und R³ für Wasserstoff stehen, sind ebenfalls besonders bevorzugt.

Auch Verbindungen gemäß der allgemeinen Formel (I), bei denen einer der Reste R² und R³ für Wasserstoff und der andere für ein Chloratom steht, sind erfindungsgemäß bevorzugt.

Erfindungsgemäß ganz besonders bevorzugte Verbindungen gemäß der Formel (I) sind ausgewählt aus 1-(1 '-Hydroxyethyl)-2,5-diaminobenzol, 1-(2'-Hydroxyethyl)-2,5-diaminobenzol, 1-(1',2'-Dihydroxyethyl)-2,5-diaminobenzol und 1-(2`-Hydroxyethyl)-4-chlor-2,5-diaminobenzol sowie den physiologisch verträglichen Salzen dieser Verbindungen.

Ebenfalls erfindungsgemäß bevorzugt sind solche p-Aminophenol-Derivate gemäß Formel (II), bei denen der Rest R⁴ für eine Methylgruppe steht.

Des weiteren sind solche erfindungsgemäßen p-Aminophenol-Derivate gemäß Formel (II) bevorzugt, bei denen die Reste R⁵ und R⁶ für Wasserstoff stehen.

Als ganz besonders bevorzugte Farbstoffkombination im Sinne der Erfindung hat sich die Kombination aus 2,4-Dichlor-3-aminophenol, 3-Methyl-4-aminophenol und einer oder mehrerer Verbindungen, ausgewählt aus 1-(1'-Hydroxyethyl)-2,5-diaminobenzol, 1-(2'-Hydroxyethyl)-2,5-diaminobenzol, 1-(1', 2'-Dihydroxyethyl)-2,5-diaminobenzol und 1-(2'-Hydroxyethyl)-4-chlor-2,5-diaminobenzol sowie den physiologisch verträglichen Salzen dieser Verbindungen, erwiesen.

Die erfindungsgemäßen farbverändernden Mittel können neben der erfindungsgemäßen Farbstoffkombination mindestens ein weiteres Farbstoffvorprodukt enthalten.

Hinsichtlich der in den erfindungsgemäßen Mitteln einsetzbaren weiteren Farbstoffvorprodukte unterliegt die vorliegende Erfindung keinerlei Einschränkungen. Die erfindungsgemäßen Mittel können als Farbstoffvorprodukte
- Oxidationsfarbstoffvorprodukte vom Entwickler- und Kuppler-Typ, und
- Vorstufen naturanaloger Farbstoffe, wie Indol- und Indolin-Derivate,
sowie Mischungen von Vertretern dieser Gruppen enthalten.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (III) 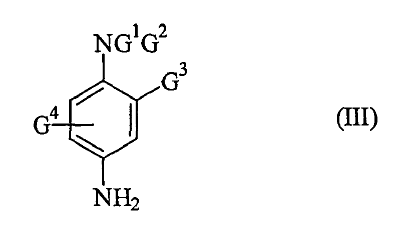 wobei
- G¹ steht für ein Wasserstoffatom, einen C₁- bis C₄-Alkykest, einen C₁- bis C₄-Monohydroxyalkylrest, einen C₂- bis C₄-Polyhydroxyalkylrest, einen (C₁- bis C₄)-Akoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, Iod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (III) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (III) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phen-ylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenylp-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (III) sind p-Phenylendiamin, p-Toluylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxygruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (IV) entsprechen, sowie ihre physiologisch verträglichen Salze: 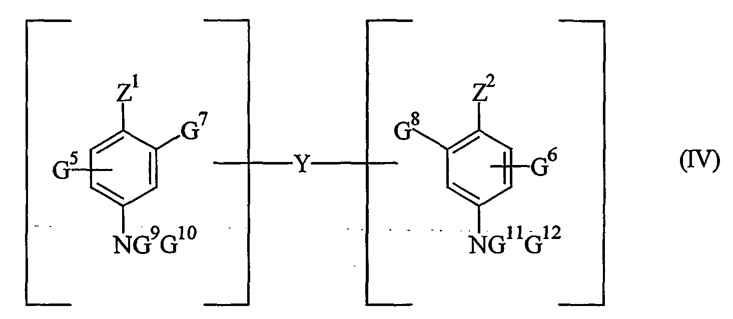 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
   mit den Maßgaben, daß
- die Verbindungen der Formel (IV) nur eine Verbrückung Y pro Molekül enthalten und
- die Verbindungen der Formel (IV) mindestens eine Aminogruppe enthalten, die mindestens ein Wasserstoffatom trägt.

Die in Formel (IV) verwendeten Substituenten sind erfindungsgemäß analog zu den obigen Ausführungen definiert.

Bevorzugte zweikernige Entwicklerkomponenten der Formel (IV) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethy-lendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piper-azin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (IV) sind N,N'-Bis-(β-hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (V) 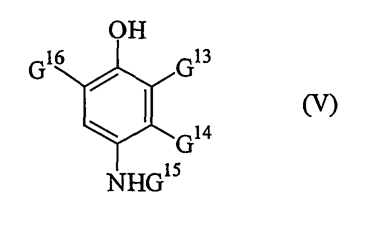 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁-bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (V) verwendeten Substituenten sind erfindungsgemäß analog zu den obigen Ausführungen definiert.

Bevorzugte p-Aminophenole der Formel (V) sind insbesondere p-Aminophenol, N-Methyl-p-aminophenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (V) sind p-Aminophenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Axnino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(β-Methoxyethyl)-amino-3-amino-6-methoxy-pyridin, 3-Amino-2-methylamino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethyl-amino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenyl-pyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropyl-pyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-me-thylpyrazol.

Bevorzugte Pyrazol-Pyrimidin-Derivate sind insbesondere die Derivate des Pyrazol-[1,5-a]-pyrimidin der folgenden Formel (VI) und dessen tautomeren Formen, sofern ein tautomeres Gleichgewicht besteht: 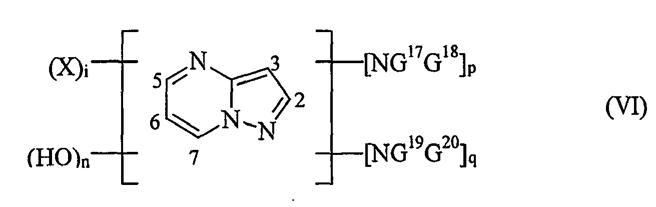 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂-bis C₄-Polyhydroxyalkylrest einen (C₁- bis C₄)-Alkoxy-(C₁- bis C₄)-alkylrest, einen C₁-bis C₄-Aminoalkylrest, der gegebenenfalls durch ein Acetyl-Ureid- oder einen SulfonylRest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁-bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkykest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (VI) verwendeten Substituenten sind erfindungsgemäß analog zu den obigen Ausführungen definiert.

Wenn das Pyrazol-[1,5-a]-pyrimidin der obenstehenden Formel (VI) eine Hydroxygruppe an einer der Positionen 2, 5 oder 7 des Ringsystems enthält, besteht ein tautomeres Gleichgewicht, das zum Beispiel im folgenden Schema dargestellt wird:

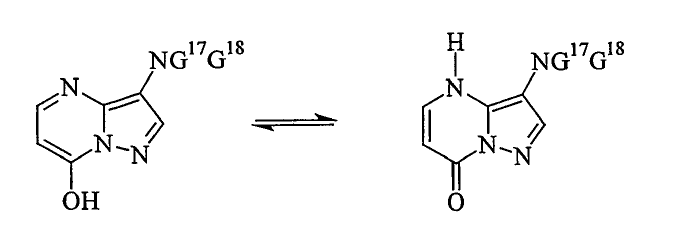

Unter den Pyrazol-[1,5-a]-pyrimidinen der obenstehenden Formel (VI) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-AmBopyrazol-[1,5-a]-pyrimidin-7-ol;
- 3-AmBopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrinlidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (VI) können wie in der Literatur beschrieben durch Zyklisierung ausgehend von einem Aminopyrazol oder von Hydrazin hergestellt werden.

Erfindungsgemäß ganz besonders bevorzugte weitere Entwicklerkomponenten sind ausgewählt aus p-Phenylendiamin, p-Toluylendiamin, p-Aminophenol, o-Aminophenol, N,N-Bis-(2'-hydroxyethyl)-p-phenylendiamin, 2-(2' ,5' -Diaminophenoxy)-ethanol, 2-Hydroxymethylamino-4-aminophenol, Bis-(4-aminophenyl)amin, 4-Amino-3-fluorphenol, 4-Amino-2-((diethylamino)-methyl)-phenol, 2-Aminomethyl-4-aminophenol, 2-Hydroxymethyl-4-aminophenol, 4-Amino-2-(2`-hydroxyethoxy)-phenol, Bis-(2-hydroxy-5 -aminophenyl)-methan, 1,4-Bis-(4`-aminophenyl)-diazacycloheptan, N,N'-Bis-(2'-hydroxyethyl)-N,N'-bis-(4'-aminophenyl)-1,3-diamino-propan-2-01,1,10-Bis-(2',5'-diaminophenyl)-1,4,7,10-tetraoxadecan,2,4,5,6-Tetraaminopyrimidin,2-Hydroxy-4,5,6-triaminopyrimidin und 4-Hydroxy-2,5,6-triaminopyrimidin, 2,4-Dihydroxy-5,6-diaminopyrimidin, 2-Dimethylamino-4,5,6-triaminopyrimidin und den Diaminopyrazol-Derivaten nach EP 0 740 931 bzw. WO 94/08970 wie beispielsweise 4,5-Diamino-1-C4'-chlorbenzyl)pyrazol, 4,5-Diamino-1-(2'-hydroxyethyl)-pyrazol sowie deren physiologisch verträglichen Salzen.

Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind:
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoracetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, 1,3-Dihydroxy-5-(methylamino)-benzol und 3-Ethylamino-4-methylphenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1,3-Bis-(2',4'-diaminophenyl)-propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxy-benzol.

Besonders bevorzugte weitere Kupplerkomponenten sind 1-Naphthol, 1,5-, 2,7- und 1,7-Dihydroxynaphthalin, m-Aminophenol, 5-Amino-2-methylphenol, 5-(2`-Hydroxyethylamino)-3-methylphenol, 2-Chlor-6-methyl-3-aminophenol, 2-Methyl-4-chlor-5-aminophenol, Resorcin, Resorcinmonomethylether, m-Phenylendiamin, 2-Amino-2-hydroxypyridin, 2-Chlorresorcin, 4-Chlorresorcin, 2-Methylresorcin, 5-Methylresorcin, 2,5-Dimethykesorcin, m-Phenylendiamin, 2-Amino-2-hydroxypyridin, 2-Methylamino-3-amino-6-methoxypyridin, 3,4-Dimethylpyridin 2,6-Dihydroxy-3,4-dimethylpyridin, 1-Phenyl-3-methyl-pyrazolon-5, 1,3- Bis-(2' ,4' -diaminophenoxy)-propan, 2,4-Diaminophenoxyethanol, 2-Amino-4-(2'-hydroxyethyl)amino-anisol, 6-Hydroxyindol sowie deren physiologisch verträglichen Salze.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die Entwicklerkomponenten sind in den erfindungsgemäßen Mitteln bevorzugt in Mengen von 0,005 bis 20 Gew.-%, vorzugsweise 0,1 bis 5 Gew.-% und die Kupplerkomponenten sind bevorzugt in Mengen von 0,005 bis 20 Gew.-%, vorzugsweise 0,1 bis 5 Gew.-%, jeweils bezogen auf das gesamte Mittel, enthalten.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (VII), 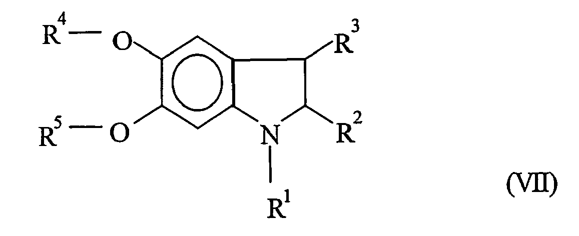 in der unabhängig voneinander
R¹ steht für Wasserstoff, eine C₁-C₄-Alkylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (VIII), 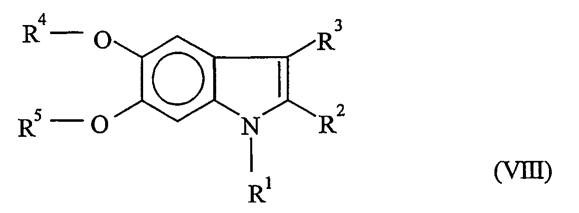 in der unabhängig voneinander
R¹ steht für Wasserstoff, eine C₁-C₄-Allcylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.
Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.%, vorzugsweise 0,2-5 Gew.-% enthalten.

Insbesondere bei der Verwendung von Farbstoff-Vorstufen vom Indolin- oder Indol-Typ hat es sich als vorteilhaft erwiesen, als Alkalisierungsmittel eine Aminosäure und/oder ein Oligopeptid einzusetzen.

Neben den Farbstoffvorprodukten können die erfindungsgemäßen Mittel zur weiteren Nuancierung direktziehende Farbstoffe enthalten. Diese sind üblicherweise ausgewählt aus Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Die erfindungsgemäßen Mittel enthalten Farbstoffvorprodukte bevorzugt in einem geeigneten wäßrigen, alkoholischen oder wäßrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tablettenförmige Formulierung zu integrieren.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist.

Als Oxidationsmittel kommen Persulfate, Chlorite und insbesondere Wasserstoffperoxid oder dessen Anlagerungsprodukte an Harnstoff, Melamin sowie Natriumborat in Frage. Man kann aber die Oxidationsfärbemittel auch zusammen mit einem Katalysator auf das Haar aufbringen, der die Oxidation der Farbstoffvorprodukte, z. B. aktiviert. Solche Katalysatoren sind z. B. Übergangsmetallverbindungen, Iodide, Chinone oder bestimmte Enzyme. Geeignete Enzyme sind z. B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z. B.
- Pyranose-Oxidase und z. B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärrbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die erfindungsgemäßen Mittel können weiterhin alle für solche Zubereitungen bekannten Wirk-, Zusatz- und Hilfsstoffe enthalten. In vielen Fällen enthalten diese Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel

R¹O-(Z)ₓ.

Diese Verbindungen sind durch die folgenden Parameter gekennzeichnet:
Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.
Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.
Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethyl-ammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammonium-chlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).
Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, - hydroxide oder -alkoholate als Katalysatoren verwendet werden. Die Verwendung von Produkten mit eingeengter Homologenverteilung kann bevorzugt sein.

Weiterhin können die erfindungsgemäßen Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.

Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat^{®}100 (Poly(dimethyldiallylammoniumchlorid)), Merquat^{®}550 (Dimethyldiallylammoniumchlorid-Acrylamid-Copolymer) und Merquat^{®} 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden.
- quaternierter Polyvinylalkohol
   sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline. Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyltrimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessemde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein zweiter Gegenstand der Erfindung ist ein Verfahren zum Färben von Keratinfasern, bei dem ein erfindungsgemäßes Mittel auf die zu färbenden Fasern aufgebracht und nach einer Einwirkungszeit wieder ausgespült und gegebenenfalls mit einem Shampoo ausgewaschen wird.

Ein dritter Gegenstand der Erfindung ist die Verwendung eines erfindungsgemäßen Mittels zur Färbung von Keratinfasern.

Die folgenden Beispiele sollen den Gegenstand der vorliegenden Anmeldung verdeutlichen ohne in jedoch hierauf zu beschränken.

### Beispiele

Es wurden die folgenden Färbezusammensetzungen hergestellt:

### Färbecreme 1 :

| | |
|---|---|
| Fettalkoholgemisch C₁₂- C₁₈ | 9,5 |
| Eumulgin^{®} B 1 | 0,4 |
| Eumulgin^{®} B2 | 0,4 |
| Akyposoft^{®} 45 NV | 8,0 |
| Texapon^{®} K 14S 70C | 3,5 |
| Plantaren^{®} 1200 | 2,5 |
| Eutanol^{®} G | 1,0 |
| Cetiol^{®} OE | 0,5 |
| Cetiol^{®} S | 0,2 |
| Arginin | 0,8 |
| Ammoniumsulfat | 0,4 |
| Natriummetabisulfit | 0,3 |
| EDTA | 0,1 |
| Ascorbinsäure | 0,2 |
| Mirapol^{®} A 15 | 3,0 |
| 1-(2'-Hydroxyethyl)-2,5-diaminobenzolsulfat | 0,12 |
| 3-Methyl-4-aminophenol | 0,009 |
| 2,4-Dichlor-3-aminophenol | 0,010 |
| Resorcin | 0,060 |
| 4-Chlorresorcin | 0,060 |
| 2-Amino-3-hydroxypyridin | 0,005 |
| 5-Amino-2-methylphenol | 0,002 |
| Wasser | ad 100 |
| Mit Ammoniaklösung auf pH 9,5 - 10 | |

### Färbecreme 2:

| | |
|---|---|
| Fettalkoholgemisch C₁₂ - C₁₈ | 9,5 |
| Eumulgin^{®} B1 | 0,4 |
| Eumulgin^{®} B2 | 0,4 |
| Akyposoft^{®} 45 NV | 8,0 |
| Texapon^{®} K 14S 70C | 3,5 |
| Plantaren^{®} 1200 | 2,5 |
| Eutanol^{®} G | 1,0 |
| Cetiol^{®} OE | 0,5 |
| Cetiol^{®} S | 0,2 |
| Arginin | 0,8 |
| Ammoniumsulfat | 0,4 |
| Natriumetabisulfit | 0,3 |
| EDTA | 0,1 |
| Ascorbinsäure | 0,2 |
| Mirapol^{®} A 15 | 3,0 |
| 1-(2'-Hydroxyethyl)-2,5-diaminobenzolsulfat | 0,82 |
| 3-Methyl-4-aminophenol | 0,46 |
| 2,4-Dichlor-3-aminophenol | 0,08 |
| Resorcin | 0,07 |
| 5-Amino-2-methylphenol | 0,55 |
| 2-Methylresorcin | 0,07 |
| 2,7-Dihydroxynaphthalin | 0,09 |
| 3-Amino-2-chlor-6-methylphenol | 0,05 |
| 4-Amino-2-nitro-diphenylamin-2-carbonsäure | 0,1 |
| Wasser | ad 100 |
| Ammoniaklösung auf pH 9,5-10 | |

### Färbecreme 3:

| | |
|---|---|
| Fettalkoholgemisch C₁₂ - C₁₈ | 9,5 |
| Eumulgin^{®} B1 | 0,4 |
| Eumulgin^{®} B2 | 0,4 |
| Akyposoft^{®} 45 NV | 8,0 |
| Texapon^{®} K 14S 70C | 3,5 |
| Plantaren® 1200 | 2,5 |
| Eutanol^{®} G | 1,0 |
| Cetiol^{®} OE | 0,5 |
| Cetiol^{®} S | 0,2 |
| Arginin | 0,8 |
| Ammoniumsulfat | 0,4 |
| Natriumetabisulfit | 0,3 |
| EDTA | 0,1 |
| Ascorbinsäure | 0,2 |
| Mirapol^{®} A 15 | 3,0 |
| 1-(2'-Hydroxyethyl)-2,5-diammobenzolsulfat | 0,7 |
| 3-Methyl-4-aminophenol | 1,1 |
| 2,4-Dichlor-3-aminophenol | 0,09 |
| Resorcin | 0,2 |
| 2-Amino-3-hydroxypyridin | 0,1 |
| 5-Amino-2-methylphenol | 0,04 |
| 2-Methylresorcin | 0,1 |
| 3-Aminophenol | 0,2 |
| 6-Nitro-1,2,3,4-tetrahydrochinoxalin | 0,15 |
| Wasser | ad 100 |
| mit Ammoniaklösung auf pH 9,5 -10. | |

### Färbecreme 4:

| | |
|---|---|
| Fettalkoholgemisch C₁₂ - C₁₈ | 9,5 |
| Eumulgin^{®} B1 | 0,4 |
| Eumulgin^{®} B2 | 0,4 |
| Akyposoft^{®} 45 NV | 8,0 |
| Texapon^{®} K 14S 70C | 3,5 |
| Plantaren^{®} 1200 | 2,5 |
| Eutanol^{®} G | 1,0 |
| Cetiol^{®} OE | 0,5 |
| Cetiol^{®} S | 0,2 |
| Arginin | 0,8 |
| Ammoniumsulfat | 0,4 |
| Natriumetabisulfit | 0,3 |
| EDTA | 0,1 |
| Ascorbinsäure | 0,2 |
| Mirapol^{®} A 15 | 3,0 |
| 1-(2' -Hydroxyethyl)-2,5-diaminobenzolsulfat | 3,92 |
| 3-Methyl-4-aminophenol | 0,2 |
| 2,4-Dichlor-3-aminophenol | 0,41 |
| Resorcin | 0,23 |
| 3-Aminophenol | 0,33 |
| 1,3-Bis-2,4-(diaminophenoxy)propan-tetrahydrochlorid | 0,66 |
| 3-Amino-2-methylamino-6-methoxypyridin | 0,13 |
| 1-Naphthol | 0,07 |
| 2-Aminophenol | 0,13 |
| Wasser | ad 100 |
| mit Ammoniaklösung auf pH 9,5-10 | |

Die Rezepturen wurden im Verhältnis 1:1 mit einer kommerziellen 6 %igen wässrigen H₂O₂-Entwicklerformulierung (Poly Diadem) gemischt und auf weißem Menschenhaar (Kerling naturweiß) im Flottenverhältnis (Verhältnis Creme zu Menschenhaar) 4:1 während 30 Minuten bei 32°C ausgefärbt.

Die Farbresultate waren wie folgt:

| Rezeptur-Nr. | Farbnuancen: |
|---|---|
| 1 | Hellaschgrau |
| 2 | Violettbraun |
| 3 | Lederbraun |
| 4 | Schwarz |

- Eumulgin^{®} B1: Cetylstearylalkohol mit ca. 12 Mol EO (INCI-Bezeichnung: Ceteareth-12) (Cognis)
- Eumulgin^{®} B2: Cetylstearylalkohol mit ca. 20 Mol EO (INCI-Bezeichnung: Ceteareth-20) (Cognis)
- Akyposoft^{®} 45 NV: C₁₂₋₁₄-Fettalkohol mit ca. 4.5 Mol EO-Essigsäure-Natrium-Salz (ca. 21% Aktivsubstanz in Wasser; INCI-Bezeichnung: Sodium Laureth-6 carboxylate) (Kao)
- Texapon^{®} K 14S 70C: Natriumlaurylmyristylethersulfat (INCI-Bezeichnung: Sodium Myreth Sulfate) (Cognis)
- Plantaren^{®} 1200: C₁₂-₁₆-Fettalkohol-1.4-glukosid (ca. 50-53% Aktivsubstanz in Wasser; INCI-Bezeichnung: Lauryl Glucoside) (Cognis)
- Eutanol^{®} G: 2-Octyldodecanol (INCI-Bezeichnung: Octyldodecanol) (Cognis)
- Cetiol^{®} OE: Dioctylether (INCI-Bezeichnung: Dicaprylylether) (Cognis)
- Cetiol^{®} S: 1,3-Bis(2-ethylhexyl)cyclohexan (INCI-Bezeichnung: Dioctylcyclohexane) (Cognis)
- Mirapol^{®} A 15: Poly[N-(3-(dimethylammonium)propyl]-N'-[3-ethylenoxyethylen-dimethylammonium)propyl]-Harnstoff-dichlorid (INCI-Bezeichnung: Polyquaternium-2) (Rhodia)

## Patentansprüche

1. Mittel zum Färben von Keratinfasern, **dadurch gekennzeichnet, daß** es in einem zum Färben geeigneten Medium als Oxidationsfarbstoffvorprodukte eine Kombination aus
a) 2,4-Dichlor-3-aminophenol,
b) mindestens einer Verbindung gemäß Formel (I) 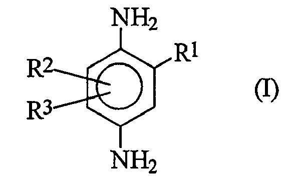 worin
- der Rest R¹ steht für eine verzweigte oder unverzweigte C₁-C₄-Mono- oder eine C₂-C₆- Polyhydroxyalkylgruppe und
- die beiden Reste R² und R³ stehen unabhängig voneinander für Wasserstoff, ein Halogenatom, eine C₁-C₄-Alkylgruppe, eine C₁-C₄-Mono- oder eine C₂-C₆-Polyhydroxyalkylgruppe, eine Amino-C₁-C₄-alkylgruppe, eine C₁-C₄-Alkoxygruppe, eine Hydroxy-C₁-C₄-alkoxygruppe oder für eine Gruppe OZ oder SZ, worin Z eine C₁-C₄-Alkylgruppe, eine C₁-C₄-Mono- oder eine C₂-C₆-Polyhydroxyalkylgruppe bedeutet, und
c) mindestens einer Verbindung gemäß Formel (II) 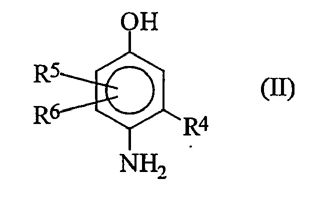 worin
- der Rest R⁴ steht für eine verzweigte oder unverzweigte C₁-C₄-Alkylgruppe und
- die beiden Reste R⁵ und R⁶ stehen unabhängig voneinander für Wasserstoff, Halogen, eine C₁-C₄-Alkylgruppe, eine C₁-C₄-Mono- oder eine C₂-C₆-Polyhydroxyalkylgruppe, eine Amino-C₁-C₄-alkylgruppe, eine C₁-C₄-Alkoxygruppe oder für eine Hydroxy-C₁-C₄-alkoxygruppe
oder den physiologisch verträglichen Salzen dieser Verbindungen enthält.

2. Mittel gemäß Anspruch 1, **dadurch gekennzeichnet, daß** der Rest R¹ für eine C₂-Monohydroxyalkylgruppe steht.

3. Mittel gemäß Anspruch 1, **dadurch gekennzeichnet, daß** der Rest R¹ für eine C₂-Dihydroxyalkylgruppe steht.

4. Mittel gemäß einem der Ansprüche 1 bis 3, **dadurch gekennzeichnet, daß** die Reste R² und R³ für Wasserstoff stehen.

5. Mittel nach einem der Ansprüche 1 bis 3, **dadurch gekennzeichnet, daß** einer der Reste R² und R³ für Wasserstoff und der andere für ein Chloratom steht.

6. Mittel nach einem der Ansprüche 1 bis 5, **dadurch gekennzeichnet, daß** die Verbindung gemäß Formel (I) ausgewählt ist aus 1-(1'-Hydroxyethyl)-2,5-diaminobenzol, 1-(2'-Hydroxyethyl)-2,5-diaminobenzol, 1-(1', 2'-Dihydroxyethyl)-2,5-diaminobenzol und 1-(2'-Hydroxyethyl)-4-chlor-2,5-diaminobenzol sowie den physiologisch verträglichen Salzen dieser Verbindungen.

7. Mittel gemäß einem der Ansprüche 1 bis 6, **dadurch gekennzeichnet, daß** der Rest R⁴ für eine Methylgruppe steht.

8. Mittel gemäß einem der Ansprüche 1 bis 7, **dadurch gekennzeichnet, daß** die Reste R⁵ und R⁶ für Wasserstoff stehen.

9. Mittel gemäß einem der Ansprüche 1 bis 8, **dadurch gekennzeichnet, daß** die erfindungsgemäße Farbstoffkombination aus 2,4-Dichlor-3-aminophenol, 3-Methyl-4-aminophenol und einer oder mehrerer Verbindungen, ausgewählt aus 1-(1'-Hydroxyethyl)-2,5-diaminobenzol, 1-(2'-Hydroxyethyl)-2,5-diaminobenzol, 1-(1', 2'-Dihydroxyethyl)-2,5-diaminobenzol und 1-(2'-Hydroxyethyl)-4-chlor-2,5-diaminobenzol sowie den physiologisch verträglichen Salzen dieser Verbindungen, ausgewählt ist.

10. Mittel nach einem der Ansprüche 1 bis 9, **dadurch gekennzeichnet, daß** es mindestens eine weitere Entwicklerkomponente enthält.

11. Mittel nach Anspruch 10, **dadurch gekennzeichnet, daß** die weitere Entwicklerkomponente ausgewählt ist aus p-Phenylendiamin, p-Toluylendiamin, p-Aminophenol, o-Aminophenol, N,N-Bis-(2'-hydroxyethyl)-p-phenylendiamin, 2-(2`,5`-Diaminophenoxy)-ethanol, 2-Hydroxymethylamino-4-aminophenol, Bis-(4-aminophenyl)amin, 4-Amino-3-fluorphenol, 4-Amino-2-((diethylamino)-methyl)-phenol, 2-Aminomethyl-4-aminophenol, 2-Hydroxymethyl-4-aminophenol, 4-Amino-2-(2`-hydroxyethoxy)-phenol, Bis-(2-hydroxy-5-aminophenyl)-methan, 1,4-Bis-(4`-aminophenyl)-diazacycloheptan, N,N'-Bis-(2'-hydroxyethyl)-N,N'-bis-(4'-aminophenyl)-1,3- diamino-propan-2-ol, 1,10-Bis-(2`,5`-diaminophenyl)-1,4,7,10-tetraoxadecan, 2,4,5,6-Tetraaminopyrimidin, 2-Hydroxy-4,5,6-triaminopyrimidin und 4-Hydroxy-2,5,6-triaminopyrimidin, 2,4-Dihydroxy-5,6-diaminopyrimidin, 2-Dimethylamino-4,5,6-triaminopyrimidin, 4,5-Diamino-1-(2`-hydroxyethyl)-pyrazol und 4,5-Diamino-1-(4`-chlorbenzyl)pyrazol sowie deren physiologisch verträglichen Salzen.

12. Mittel nach einem der Ansprüche 1 bis 11, **dadurch gekennzeichnet, daß** es mindestens eine weitere Kupplerkomponente enthält.

13. Mittel nach Anspruch 12, **dadurch gekennzeichnet, daß** die weitere Kupplerkomponente ausgewählt ist aus 1-Naphthol, 1,5-, 2,7- und 1,7-Dihydroxynaphthalin, m-Aminophenol, 5-Amino-2-methylphenol, 5-(2'-Hydroxyethylamino)-3-methylphenol, 2-Chlor-6-methyl-3-aminophenol, 2-Methyl-4-chlor-5-aminophenol, Resorcin, Resorcinmonomethylether, m-Phenylendiamin, 2-Chlorresorcin, 4-Chlorresorcin, 2-Methylresorcin, 5-Methylresorcin, 2,5-Dimethylresorcin, m-Phenylendiamin, 2-Amino-3-hydroxy-pyridin, 2-Methylamino-3-amino-6-methoxypyridin, 3,4-Dimethylpyridin, 2,6-Dihydroxy-3,4-dimethylpyridin, 1-Phenyl-3-methyl-pyrazolon-5, 1,3-Bis-(2',4'-diaminophenoxy)-propan, 2,4-Diaminophenoxyethanol 2-Amino-4-(2'-hydroxyethyl)amino-anisol, 6-Hydroxyindol sowie deren physiologisch verträglichen Salzen.

14. Mittel nach einem der Ansprüche 1 bis 13, **dadurch gekennzeichnet, daß** es weiterhin Vorstufen naturanaloger Farbstoffe, wie Indol- und Indolin-Derivate, enthält.

15. Mittel nach einem der Ansprüche 1 bis 14, **dadurch gekennzeichnet, daß** Entwicklerkomponeten in einer Menge von 0,005 bis 20 Gew.-%, vorzugsweise 0,1 bis 5 Gew.- %, und Kupplerkomponenten in einer Menge von 0,005 bis 20 Gew.-%, vorzugsweise 0,1 bis 5 Gew.-%, jeweils bezogen auf das gesamte Oxidationsfärbemittel, enthalten sind.

16. Mittel nach einem der Ansprüche 1 bis 15, **dadurch gekennzeichnet, daß** weiterhin mindestens ein direktziehender Farbstoff enthalten ist.

17. Verfahren zum Färben von Keratinfasern, **dadurch gekennzeichnet, daß** ein Mittel zum Färben von Keratinfasern nach einem der Ansprüche 1 bis 16 auf die zu färbenden Fasern aufgebracht und nach einer Einwirkungszeit wieder ausgespült und gegebenenfalls mit einem Shampoo ausgewaschen wird.

18. Verwendung eines Mittels gemäß den Ansprüchen 1 bis 16 zur Färbung von Keratinfasern.

## Claims

1. Composition for colouring keratin fibres, **characterized in that** it comprises, in a medium suitable for the colouring, as oxidation dye precursors, a combination of
a) 2,4-dichloro-3-aminophenol,
b) at least one compound according to formula (I) 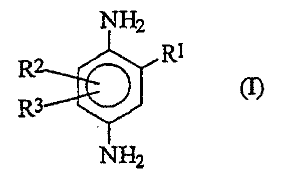 in which
- the radical R¹ is a branched or unbranched C₁-C₄-mono- or a C₂-C₆-polyhydroxyalkyl group and
- the two radicals R² and R³, independently of one another, are hydrogen, a halogen atom, a C₁-C₄-alkyl group, a C₁-C₄-mono- or a C₂-C₆-polyhydroxyalkyl group, an amino-C₁-C₄-alkyl group, a C₁-C₄-alkoxy group, a hydroxy-C₁-C₄-alkoxy group or a group OZ or SZ, in which Z is a C₁-C₄-alkyl group, a C₁-C₄-mono- or a C₂-C₆-polyhydroxyalkyl group, and
c) at least one compound according to formula (II) 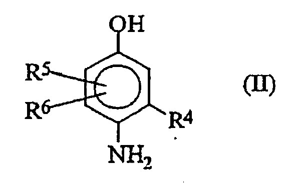 in which
- the radical R⁴ is a branched or unbranched C₁-C₄-alkyl group and
- the two radicals R⁵ and R⁶, independently of one another, are hydrogen, halogen, a C₁-C₄-alkyl group, a C₁-C₄-mono- or a C₂-C₆-polyhydroxyalkyl group, an amino-C₁-C₄-alkyl group, a C₁-C₄-alkoxy group or a hydroxy-C₁-C₄-alkoxy group
or the physiologically compatible salts of these compounds.

2. Composition according to Claim 1, **characterized in that** the radical R¹ is a C₂-monohydroxyalkyl group.

3. Composition according to Claim 1, **characterized in that** the radical R¹ is a C₂-dihydroxyalkyl group.

4. Composition according to one of Claims 1 to 3, **characterized in that** the radicals R² and R³ are hydrogen.

5. Composition according to one of Claims 1 to 3, **characterized in that** one of the radicals R² and R³ is hydrogen and the other is a chlorine atom.

6. Composition according to one of Claims 1 to 5, **characterized in that** the compound according to formula (I) is selected from 1-(1'-hydroxyethyl) -2,5-diaminobenzene, 1-(2'-hydroxyethyl)-2,5-diaminobenzene, 1-(1',2'-dihydroxyethyl)-2,5-diaminobenzene and 1-(2'-hydroxyethyl)-4-chloro-2,5-diaminobenzene, and the physiologically compatible salts of these compounds.

7. Composition according to one of Claims 1 to 6, **characterized in that** the radical R⁴ is a methyl group.

8. Composition according to one of Claims 1 to 7, **characterized in that** the radicals R⁵ and R⁶ are hydrogen.

9. Composition according to one of Claims 1 to 8, **characterized in that** the dye combination according to the invention is selected from 2,4-dichloro-3-aminophenol, 3-methyl-4-aminophenol and one or more compounds selected from 1-(1'-hydroxyethyl)-2,5-diaminobenzene, 1-(2'-hydroxyethyl)-2,5-diaminobenzene, 1-(1',2'-dihydroxyethyl)-2,5-diaminobenzene and 1-(2'-hydroxyethyl)-4-chloro-2,5-diaminobenzene, and the physiologically compatible salts of these compounds.

10. Composition according to one of Claims 1 to 9, **characterized in that** it comprises at least one further developer component.

11. Composition according to Claim 10, **characterized in that** the further developer component is selected from p-phenylenediamine, p-tolylenediamine, p-aminophenol, o-aminophenol, N,N-bis(2'-hydroxyethyl)-p-phenylenediamine, 2-(2',5'-diaminophenoxy)ethanol, 2-hydroxymethylamino-4-aminophenol, bis(4-aminophenol)amine, 4-amino-3-fluorophenol, 4-amino-2-((di-ethylamino)methyl)phenol, 2-aminomethyl-4-aminophenol, 2-hydroxymethyl-4-aminophenol, 4-amino-2-(2'-hydroxy-ethoxy)phenol, bis(2-hydroxy-5-aminophenol)methane, 1,4-bis (4'-aminophenyl) diazacycloheptane, N,N*'*-bis (2'-hydroxyethyl)-N,N'-bis (4'-aminophenyl)-1,3-diamino-propan-2-ol, 1,10-bis(2',5'-diaminophenyl)-1,4,7,10-tetraoxadecane, 2,4,5,6-tetraaminopyrimidine, 2-hydroxy-4,5,6-triaminopyrimidine and 4-hydroxy-2,5,6-triaminopyrimidine, 2,4-dihydroxy-5,6-diaminopyrimidine, 2-dimethylamino-4,5,6-triaminopyrimidine, 4,5-diamino-1-(2'-hydroxyethyl)pyrazole and 4,5-diamino-1-(4'-chlorobenzyl)pyrazole, and physiologically compatible salts thereof.

12. Composition according to one of Claims 1 to 11, **characterized in that** it comprises at least one further coupler component.

13. Composition according to Claim 12, **characterized in that** the further coupler component is selected from 1-naphthol, 1,5-, 2,7- and 1,7-dihydroxynaphthalene, m-aminophenol, 5-amino-2-methylphenol, 5-(2'-hydroxy-ethylamino)-3-methylphenol, 2-chloro-6-methyl-3-aminophenol, 2-methyl-4-chloro-5-aminophenol, resorcinol, resorcinol monomethyl ether, m-phenylenediamine, 2-chlororesorcinol, 4-chlororesorcinol, 2-methylresorcinol, 5-methylresorcinol, 2,5-dimethylresorcinol, m-phenylenediamine, 2-amino-3-hydroxypyridine, 2-methylamino-3-amino-6-methoxypyridine, 3,4-dimethylpyridine, 2,6-dihydroxy-3,4-dimethylpyridine, 1-phenyl-3-methylpyrazolone-5,1,3-bis (2',4'-diamino-phenoxy)propane, 2,4-diaminophenoxyethanol, 2-amino-4-(2'-hydroxyethyl)aminoanisole, 6-hydroxyindole, and physiologically compatible salts thereof.

14. Composition according to one of Claims 1 to 13, **characterized in that** it further comprises precursors of nature-analogous dyes, such as indole derivatives and indoline derivatives.

15. Composition according to one of Claims 1 to 14, **characterized in that** developer components are present in an amount of from 0.005 to 20% by weight, preferably 0.1 to 5% by weight, and coupler components are present in an amount of from 0.005 to 20% by weight, preferably 0.1 to 5% by weight, in each case based on the total oxidation colorant.

16. Composition according to one of Claims 1 to 15, **characterized in that** furthermore at least one direct dye is present.

17. Method for colouring keratin fibres, **characterized in that** a composition for colouring keratin fibres according to one of Claims 1 to 16 is applied to the fibres to be coloured and, after a contact time, is rinsed out again and is optionally washed out with a shampoo.

18. Use of a composition according to Claims 1 to 16 for colouring keratin fibres.

## Revendications

1. Agent pour teindre les fibres kératiniques, **caractérisé en ce qu'**il contient dans un milieu convenant à la teinture, en tant que produit précurseur de colorant par oxydation, une combinaison
a) de 2,4-dichloro-3-aminophénol,
b) d'au moins un composé selon la formule (I) 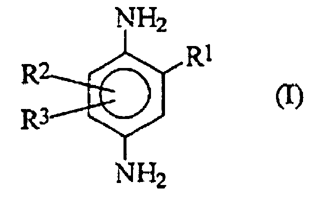 dans laquelle
- le radical R¹ représente un groupe monohydroxyalkyle en C₁ à C₄ ou un groupe polyhydroxyalkyle en C₂ à C₆, ramifié ou non ramifié, et
- les deux radicaux R² et R³ représentent indépendamment l'un de l'autre, un atome d'hydrogène, un atome d'halogène, un groupe alkyle en C₁ à C₄, un groupe monohydroxyalkyle en C₁ à C₄ ou un groupe polyhydroxyalkyle en C₂ à C₆, un groupe aminoalkyle en C₁ à C₄, un groupe alcoxy en C₁ à C₄, un groupe hydroxyalcoxy en C₁ à C₄ ou un groupe OZ ou SZ, dans lequel Z désigne un groupe alkyle en C₁ à C₄, un groupe monohydroxyalkyle en C₁ à C₄ ou un groupe polyhydroxyalkyle en C₂ à C₆, et
c) d'au moins un composé selon la formule (II) 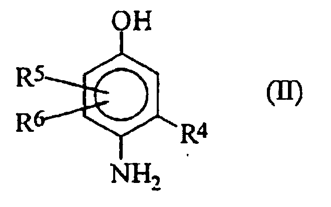 dans laquelle
- le radical R⁴ représente un groupe alkyle en C₁ à C₄, ramifié ou non ramifié, et
- les deux radicaux R⁵ et R⁶ représentent indépendamment l'un de l'autre, un atome d'hydrogène, un atome d'halogène, un groupe alkyle en C₁ à C₄, un groupe monohydroxyalkyle en C₁ à C₄ ou un groupe polyhydroxyalkyle en C₂ à C₆, un groupe aminoalkyle en C₁ à C₄, un groupe alcoxy en C₁ à C₄ ou un groupe hydroxyalcoxy en C₁ à C₄
ou des sels acceptables sur le plan physiologique de ces composés.

2. Agent selon la revendication 1, **caractérisé en ce que** le radical R¹ représente un groupe monohydroxyalkyle en C₂.

3. Agent selon la revendication 1, **caractérisé en ce que** le radical R¹ représente un groupe dihydroxyalkyle en C₂.

4. Agent selon l'une quelconque des revendications 1 à 3, **caractérisé en ce que** les radicaux R² et R³ représentent un atome d'hydrogène.

5. Agent selon l'une quelconque des revendications 1 à 3, **caractérisé en ce que** l'un des radicaux R² et R³ représente un atome d'hydrogène et l'autre représente un atome de chlore.

6. Agent selon l'une quelconque des revendications 1 à 5, **caractérisé en ce que** le composé selon la formule (1) est choisi parmi le 1 -(1'-hydroxyéthyl)-2,5-diaminobenzène, le 1-(2'-hydroxyéthyl)-2,5-diamino-benzène, le 1-(1',2'-dihydroxyéthyl)-2,5-diaminobenzène, et le 1-(2'-hydroxy-éthyl)-4-chloro-2,5-diaminobenzène, ainsi que les sels acceptables sur le plan physiologique de ces composés.

7. Agent selon l'une quelconque des revendications 1 à 6, **caractérisé en ce que** le radical R⁴ représente un groupe méthyle.

8. Agent selon l'une quelconque des revendications 1 à 7, **caractérisé en ce que** les radicaux R⁵ et R⁶ représentent un atome d'hydrogène.

9. Agent selon l'une quelconque des revendications 1 à 8, **caractérisé en ce que** la combinaison selon l'invention de colorant est choisie parmi le 2,4-dichloro-3-aminophénol, le 3-méthyl-4-aminophénol et un ou plusieurs composés choisis parmi le 1-(1'-hydroxyéthyl)-2,5-diaminobenzène, le 1-(2'-hydroxyéthyl)-2,5-diaminobenzène, le 1-(1',2'-dihydroxyéthyl)-2,5-diamino-benzène, et le 1-(2'-hydroxyéthyl)-4-chloro-2,5-diaminobenzène, ainsi que les sels acceptables sur le plan physiologique de ces composés.

10. Agent selon l'une quelconque des revendications 1 à 9, **caractérisé en ce qu'**il contient au moins un composant révélateur supplémentaire.

11. Agent selon la revendication 10, **caractérisé en ce que** le composant révélateur supplémentaire est choisi parmi la p-phénylène-diamine, la p-toluylène-diamine, le p-aminophénol, l'o-aminophénol, la N,N-bis-(2'-hydroxyéthyl)-p-phénylènediamine, le 2-(2',5'-diaminophénoxy)éthanol, le 2-hydroxyméthylamino-4-aminophénol, la bis-(4-aminophényl)amine, le 4-amino-3-fluorophénol, le 4-amino-2-((diéthylamino)-méthyl)-phénol, le 2-aminométhyl-4-aminophénol, le 2-hydroxyméthyl-4-aminophénol, le 4-amino-2-(2'-hydroxyéthoxy)-phénol, le bis-(2-hydroxy-5-aminophényl)-méthane, le 1,4-bis-(4'-aminophényl)-diazacycloheptane, le N,N'-bis-(2'-hydroxyéthyl)-N,N'-bis-(4'-aminophényl)-1,3-diaminopropan-2-ol, le 1,10-bis-(2',5'-diaminophényl)-1,4,7,10-tétraoxadécane, la 2,4,5,6-tétraaminopyrimidine, la 2-hydroxy-4,5,6-triaminopyrimidine, et la 4-hydroxy-2,5,6-triaminopyrimidine, la 2,4-dihydroxy-5,6-diaminopyrimidine, la 2-diméthylamino-4,5,6-triaminopyrimidine, le 4,5-diamino-1-(2'-hydroxyéthyl)-pyrazole et le 4,5-diamino-1-(4'-chlorobenzyl)-pyrazole ainsi que leurs sels acceptables sur le plan physiologique.

12. Agent selon l'une quelconque des revendications 1 à 11, **caractérisé en ce qu'**il contient au moins un composant de couplage supplémentaire.

13. Agent selon la revendication 12, **caractérisé en ce que** le composant de couplage supplémentaire est choisi parmi le 1-napthol, le 1,5-, 2,7- et 1,7-dihydroxynaphtalène, le m-aminophénol, le 5-amino-2-méthylphénol, le 5-(2'-hydroxyéthylamino)-3-méthylphénol, le 2-chloro-6-méthyl-3-aminophénol, le 2-méthyl-4-chloro-5-aminophénol, le résorcinol, l'éther mono-méthylique de résorcinol, la m-phénylènediamine, le 2-chlororésorcinol, le 4-chlororésorcinol, le 2-méthyl-résorcinol, le 5-méthylrésorcinol, le 2,5-diméthyl-résorcinol, la m-phénylènediamine, la 2-amino-3-hydroxypyridine, la 2-méthyl-amino-3-amino-6-méthoxypyridine, la 3,4-diméthylpyridine, la 2,6-dihydroxy-3,4-diméthylpyridine, la 1-phényl-3-méthyl-pyrazolone-5, le 1,3-bis-(2',4'-diaminophénoxy)-propane, le 2,4-diaminophénoxyéthanol, le 2-amino-4(2'-hydroxyéthyl)amino-anisole, le 6-hydroxyindole ainsi que leurs sels acceptables sur le plan physiologique.

14. Agent selon l'une quelconque des revendications 1 à 13, **caractérisé en ce qu'**il contient en outre des précurseurs de colorants analogues à ceux de la nature, comme les dérivés d'indole et d'indoline.

15. Agent selon l'une quelconque des revendications 1 à 14, **caractérisé en ce que** des composants révélateurs sont contenus en une quantité de 0,005 à 20% en poids, de préférence de 0,1 à 5% en poids, et des composants de couplage sont contenus en une quantité de 0,05 à 20% en poids, de préférence de 0,1 à 5% en poids, à chaque fois par rapport à la teinture totale par oxydation.

16. Agent selon l'une quelconque des revendications 1 à 15, **caractérisé en ce qu'**au moins un colorant direct est contenu en plus.

17. Procédé pour teindre les fibres kératiniques, **caractérisé en ce qu'**un agent pour teindre les fibres kératiniques selon l'une quelconque des revendications 1 à 16, est appliqué sur les fibres à teindre et est rincé de nouveau après une durée d'action et éventuellement éliminé par lavage avec un shampoing.

18. Utilisation d'un agent selon les revendications 1 à 16, pour teindre les fibres kératiniques.
